# EUROPÄISCHE PATENTSCHRIFT

(11) **EP 4 198 001 B1**
(45) Veröffentlichungstag und Bekanntmachung des Hinweises auf die Patenterteilung: **22.05.2024**
(21) Anmeldenummer: 21215366.2
(22) Anmeldetag: 17.12.2021
(51) Int. Cl.: C07C 45/00, C07F 15/00

(54) **PT-BIPHENYL-IOD-KOMPLEX UND PT-BIPHENYL-BROM-KOMPLEX**
PT-BIPHENYL-IODINE-COMPLEX AND PT-BIPHENYL-BROMINE COMPLEX
COMPLEXE PT-BIPHÉNYL-IODE ET COMPLEXE PT-BIPHÉNYL-BROME

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- WO-A1-99/50214
- GB-A- 1 368 434
- Hariharasarma Maheswaran ET AL: "X-ray crystal structure of cis-{( )-6,6'-[[1,1'-biphenyl]-2,2'-diylbis(oxy)] bis-dibenzo[d,f][1,3,2]dioxaphosphepin} diiodoplatinum(II).dichloromethane", Journal of Chemical Crystallography, 1. Januar 1999 (1999-01-01), Seiten 87-91, XP055922875, Gefunden im Internet: URL:https://link.springer.com/content/pdf/ 10.1023/A:1009579516577.pdf [gefunden am 2022-05-19]

## Beschreibung

Die vorliegende Erfindung betrifft einen Pt-Biphenyl-lod-Komplex und Pt-Biphenyl-Brom-Komplex, sowie deren Verwendung zur Katalyse einer Hydroformylierungsreaktion.

In EP 2663573 B1 wird ein Verfahren zur Herstellung von (1) beschrieben.

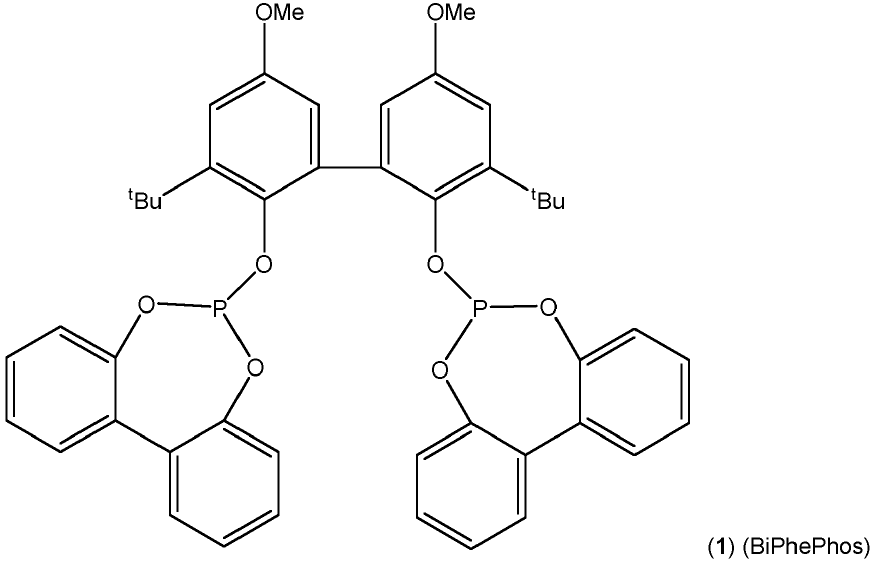

In Maheswaran Hariharasarma et al., "X-ray crystal structure of cis-{(+/-)6,6'-[[1,1'-biphenyl]-2,2'-diylbis(oxy)]bis-dibenzo[d,f][1,3,2]dioxaphosphepinjdiiodoplatinum(II)dichloromethane", Journal of Chemical Crystallography, Vol. 29, No. 1, 1999 wurden Biphenylliganden mit Hilfe von Röntgenstrukturanalyse untersucht.

In WO 99/50214 A1 wird ein Verfahren zur Carbonylierung beschrieben, bei welchem superkritisches CO₂ zum Einsatz kommt.

In GB 1 368 434 A wird ein Verfahren zur Hydroformylierung von Propylen beschrieben.

Der vorliegende Erfindung lag die Aufgabe zugrunde, einen neuen Komplex bereitzustellen. Der Komplex soll hierbei bei der Katalyse von Hydroformylierungsreaktionen eine gesteigerte Ausbeute liefern.

Diese Aufgabe wird gelöst durch einen Komplex gemäß Anspruch 1.

Komplex umfassen:
a) Pt;
b) einen Liganden entsprechend der Formel (**I**): 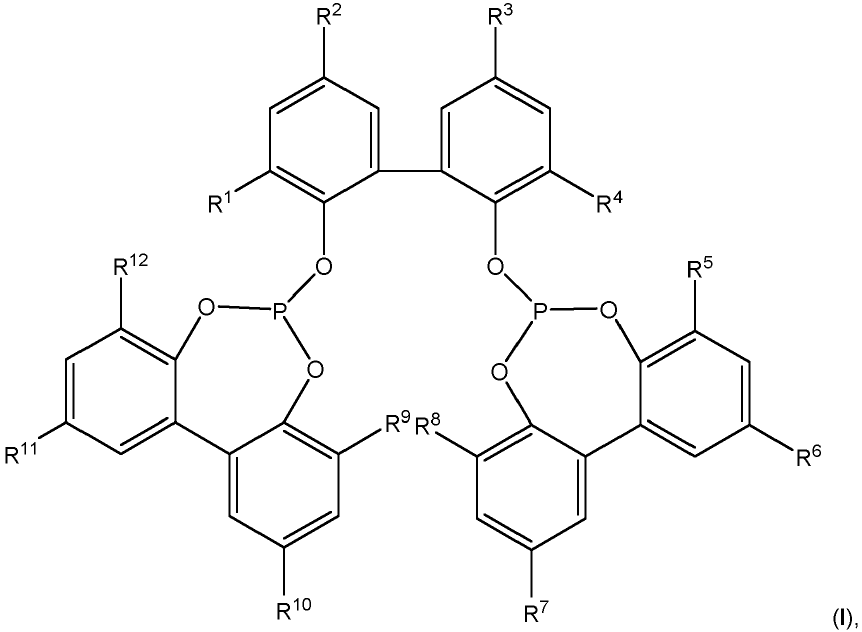 wobei R¹, R², R³, R⁴, ausgewählt sind aus: -(C₁-C₁₂)-Alkyl, -O-(C₁-C₁₂)-Alkyl und R⁵, R⁶, R⁷, R⁸, R⁹, R¹⁰, R¹¹, R¹² ausgewählt sind aus: -H, -(C₁-C₁₂)-Alkyl, -O-(C₁-C₁₂)-Alkyl, -(C₆-C₂₀)-Aryl;
c) einen lod-Liganden oder einen Brom-Liganden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Definition von (C₁-C₁₂)-Alkyl gilt analog auch für das (C₁-C₁₂)-Alkyl in -O-(C₁-C₁₂)-Alkyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform stehen R¹ und R⁴ für -(C₁-C₁₂)-Alkyl.

In einer Ausführungsform stehen R¹ und R⁴ für -'Bu.

In einer Ausführungsform stehen R² und R³ für -O-(C₁-C₁₂)-Alkyl.

In einer Ausführungsform stehen R² und R³ für -OMe.

In einer Ausführungsform stehen R⁵, R⁶, R⁷, R⁸, R⁹, R¹⁰, R¹¹, R¹² für -H.

In einer Ausführungsform stehen weist die Verbindung gemäß der Formel (**I**) die Struktur (**1**) auf:

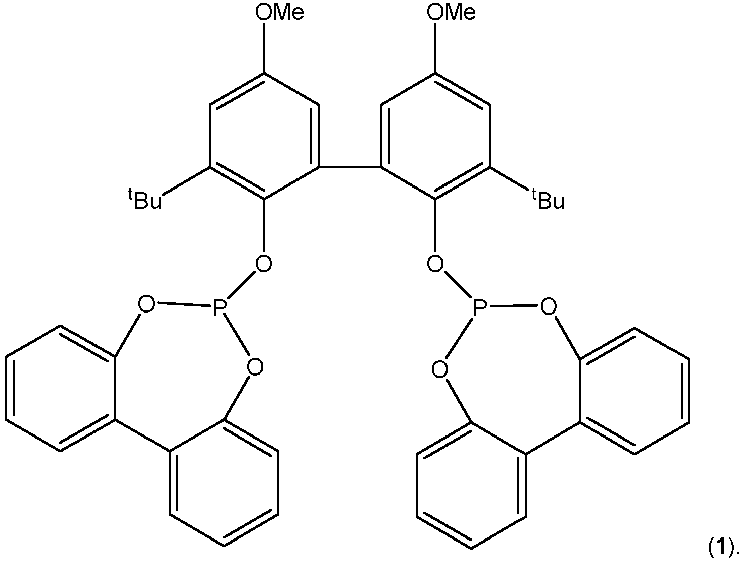

In einer Ausführungsform weist der Komplex genau einen Liganden entsprechend der Formel (**I**) auf.

In einer Ausführungsform weist der Komplex mindestens zwei lod-Liganden auf.

In einer Ausführungsform weist der Komplex genau zwei lod-Liganden auf.

In einer Ausführungsform weist der Komplex mindestens zwei Brom-Liganden auf.

In einer Ausführungsform weist der Komplex genau zwei Brom-Liganden auf.

Neben dem Komplex an sich, wird auch dessen Verwendung zur Katalyse einer Hydroformylierungsreaktion beansprucht.

Verwendung eines zuvor beschriebenen Komplexes zur Katalyse einer Hydroformylierungsreaktion.

Im Folgenden soll die Erfindung anhand von Ausführungsbeispielen näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und 1-Octen in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 20 h bei 120 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Variation des Halogens

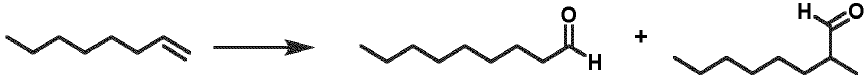

Reaktionsbedingungen:
1.0 mmol 1-Octen, 1.0 mol% PtX₂, 2.2 äquivalente Ligand (**1**), Lösungsmittel: Toluol, p(CO/H₂): 40 bar, T: 120 °C, t: 20 h.

### Ausbeuten:

| Ligand | Halogen | Ausbeute [%] |
|---|---|---|
| 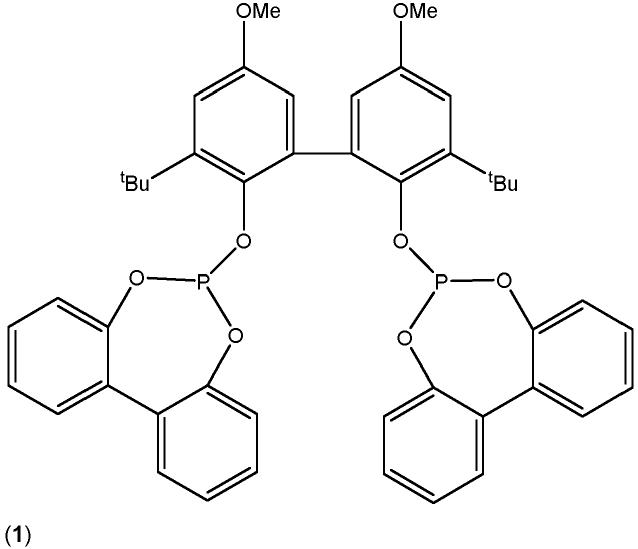 | I / Br | 69 / 27 |

Wie die Versuchsergebnisse zeigen, wird die Aufgabe durch den erfindungsgemäßen Komplex gelöst.

## Patentansprüche

1. Komplex umfassen:
a) Pt;
b) einen Liganden entsprechend der Formel (**I**): 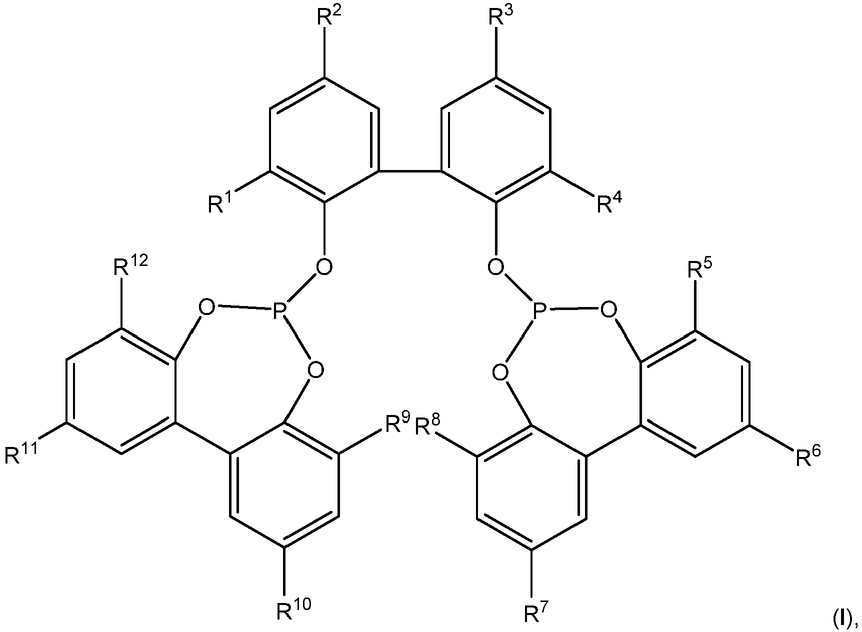
wobei R¹, R², R³, R⁴ ausgewählt sind aus: -(C₁-C₁₂)-Alkyl, -O-(C₁-C₁₂)-Alkyl und
R⁵, R⁶, R⁷, R⁸, R⁹, R¹⁰, R¹¹, R¹² ausgewählt sind aus: -H, -(C₁-C₁₂)-Alkyl, -O-(C₁-C₁₂)-Alkyl, - (C₆-C₂₀)-Aryl;
c) einen lod-Liganden oder einen Brom-Liganden.

2. Komplex nach Anspruch 1,
wobei R¹ und R⁴ für -(C₁-C₁₂)-Alkyl stehen.

3. Komplex nach einem der Ansprüche 1 bis 2,
wobei R² und R³ für -O-(C₁-C₁₂)-Alkyl stehen.

4. Komplex nach einem der Ansprüche 1 bis 3,
wobei R⁵, R⁶, R⁷, R⁸, R⁹, R¹⁰, R¹¹, R¹² für -H stehen.

5. Komplex nach einem der Ansprüche 1 bis 4,
wobei die Verbindung gemäß der Formel (**I**) die Struktur (**1**) aufweist:

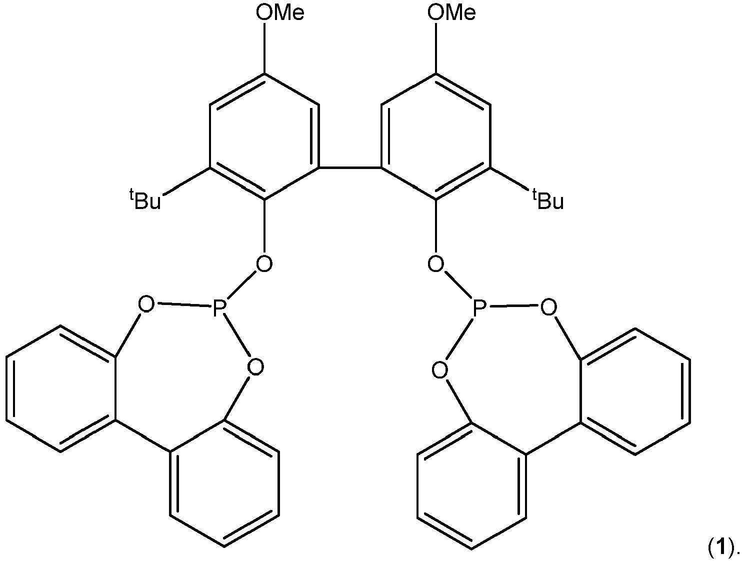

6. Komplex nach einem der Ansprüche 1 bis 5,
wobei der Komplex genau einen Liganden entsprechend der Formel (**I**) aufweist.

7. Komplex nach einem der Ansprüche 1 bis 6,
wobei der Komplex mindestens zwei lod-Liganden aufweist.

8. Komplex nach Anspruch 7,
wobei der Komplex genau zwei lod-Liganden aufweist.

9. Komplex nach einem der Ansprüche 1 bis 6,
wobei der Komplex mindestens zwei Brom-Liganden aufweist.

10. Komplex nach Anspruch 9,
wobei der Komplex genau zwei Brom-Liganden aufweist.

11. Verwendung eines Komplexes nach einem der Ansprüche 1 bis 10 zur Katalyse einer Hydroformylierungsreaktion.

## Claims

1. Complex comprising:
a) Pt;
b) a ligand corresponding to formula (I): 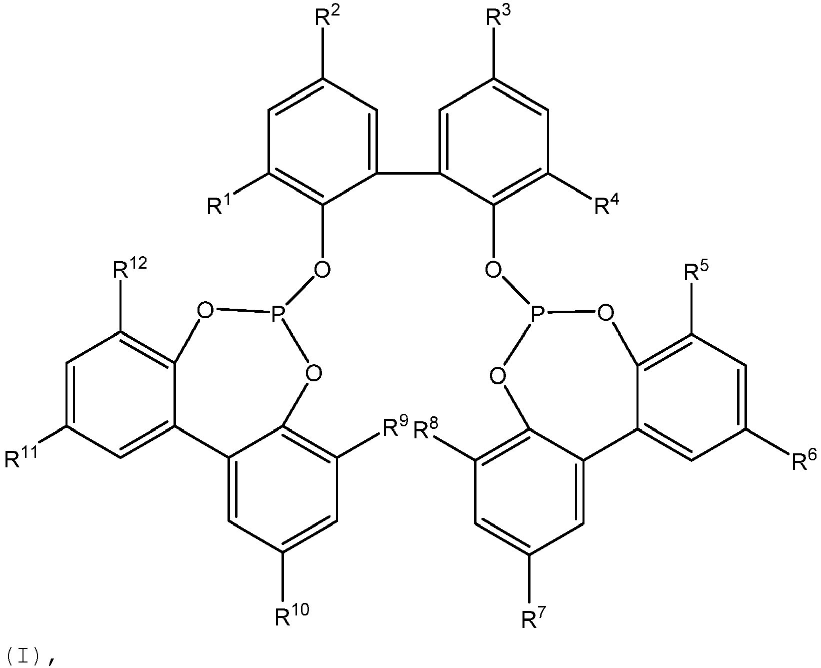
where R¹, R², R³, R⁴ are selected from: -(C₁-C₁₂)-alkyl, - O-(C₁-C₁₂)-alkyl, and
R⁵, R⁶, R⁷, R⁸, R⁹, R¹⁰, R¹¹, R¹² are selected from: -H, - (C₁-C₁₂)-alkyl, -O-(C₁-C₁₂)-alkyl, -(C₆-C₂₀)-aryl;
c) an iodine ligand or a bromine ligand.

2. Complex according to Claim 1,
where R¹ and R⁴ are -(C₁-C₁₂)-alkyl.

3. Complex according to either of Claims 1 and 2, where R² and R³ are -O-(C₁-C₁₂)-alkyl.

4. Complex according to any of Claims 1 to 3,
where R⁵, R⁶, R⁷, R⁸, R⁹, R¹⁰, R¹¹, R¹² are -H.

5. Complex according to any of Claims 1 to 4,
wherein the compound of formula (I) has the structure (1) :

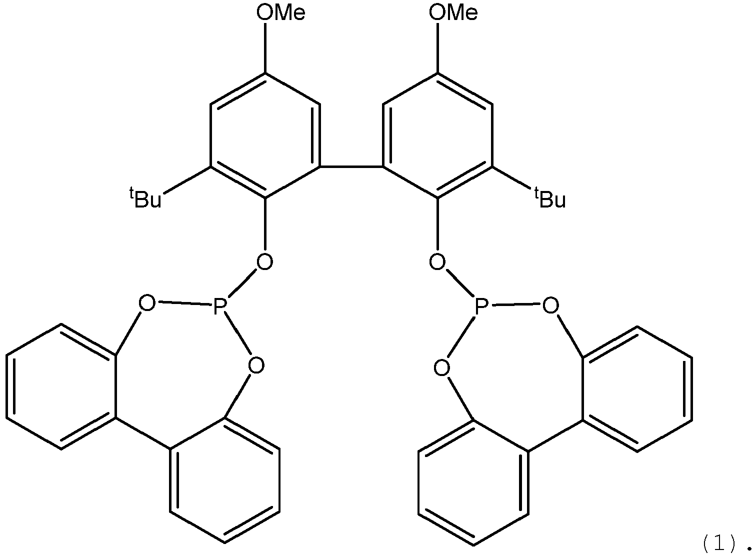

6. Complex according to any of Claims 1 to 5,
wherein the complex has exactly one ligand corresponding to formula (I).

7. Complex according to any of Claims 1 to 6,
wherein the complex has at least two iodine ligands.

8. Complex according to Claim 7,
wherein the complex has exactly two iodine ligands.

9. Complex according to any of Claims 1 to 6,
wherein the complex has at least two bromine ligands.

10. Complex according to Claim 9,
wherein the complex has exactly two bromine ligands.

11. Use of a complex according to any of Claims 1 to 10 for catalysis of a hydroformylation reaction.

## Revendications

1. Complexe comprenant :
a) Pt ;
b) un ligand de Formule (I) : 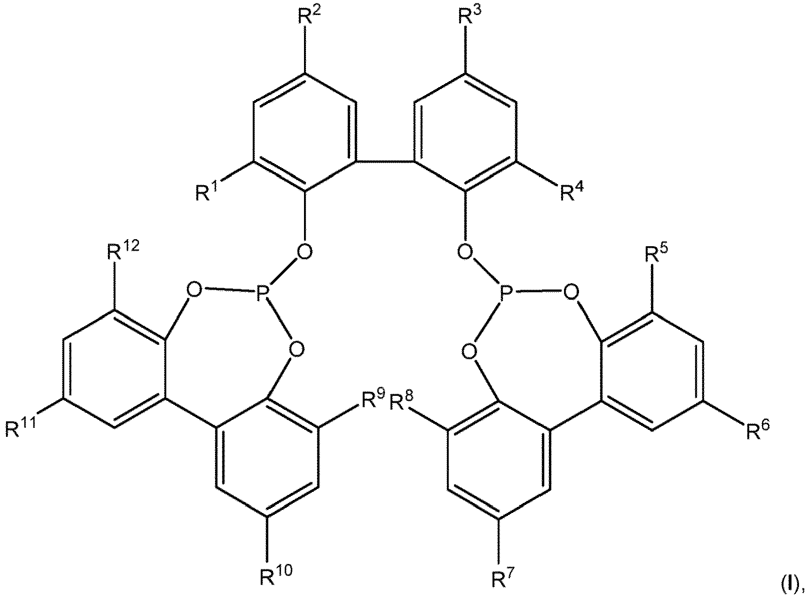
dans laquelle R¹, R², R³, R⁴ sont choisis parmi un - alkyle en C₁-C₁₂, un -O-alkyle en C₁-C₁₂ et
R⁵, R⁶, R⁷, R⁸, R⁹, R¹⁰, R¹¹, R¹² sont choisis parmi -H, - un alkyle en C₁-C₁₂, un -O-alkyle en C₁-C₁₂, un -aryle en C₆-C₂₀ ;
c) un ligand iodé ou un ligand bromé.

2. Complexe selon la revendication 1,
dans lequel R¹ et R⁴ représentent un alkyle en C₁-C₁₂.

3. Complexe selon l'une des revendications 1 à 2, dans lequel R² et R³ représentent un -O-alkyle en C₁-C₁₂.

4. Complexe selon l'une des revendications 1 à 3, dans lequel R⁵, R⁶, R⁷, R⁸, R⁹, R¹⁰, R¹¹, R¹² représentent -H.

5. Complexe selon l'une des revendications 1 à 4, dans lequel le composé de Formule (I) a la structure (1) :

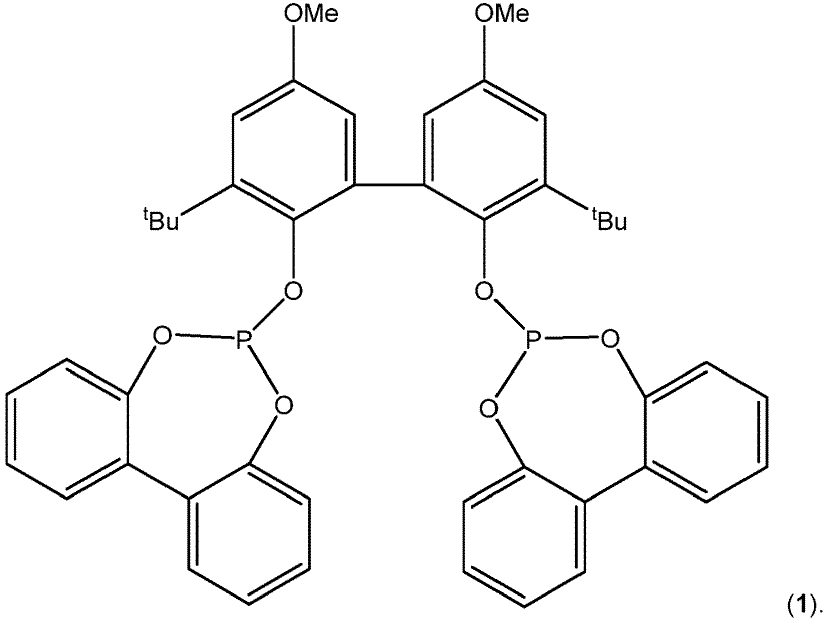

6. Complexe selon l'une des revendications 1 à 5, le complexe comprenant exactement un ligand de Formule (I) .

7. Complexe selon l'une des revendications 1 à 6, le complexe comprenant au moins deux ligands iodés.

8. Complexe selon la revendication 7, le complexe comprenant exactement deux ligands iodés.

9. Complexe selon l'une des revendications 1 à 6, le complexe comprenant au moins deux ligands bromés.

10. Complexe selon la revendication 9, le complexe comprenant exactement deux ligands bromés.

11. Utilisation d'un complexe selon l'une des revendications 1 à 10 pour la catalyse d'une réaction d'hydroformylation.
